(19) Europäisches Patentamt
European Patent Office
Office européen des brevets

(11) Veröffentlichungsnummer : **0 304 017 B1**

(12) # EUROPÄISCHE PATENTSCHRIFT

(45) Veröffentlichungstag der Patentschrift :
**16.10.91 Patentblatt 91/42**

(51) Int. Cl.$^5$ : **C07C 323/25**

(21) Anmeldenummer : **88113300.3**

(22) Anmeldetag : **17.08.88**

(54) **Verfahren zur Herstellung von Salzen des N-Acetylcysteins.**

(30) Priorität : **21.08.87 DE 3727896**

(43) Veröffentlichungstag der Anmeldung :
**22.02.89 Patentblatt 89/08**

(45) Bekanntmachung des Hinweises auf die Patenterteilung :
**16.10.91 Patentblatt 91/42**

(84) Benannte Vertragsstaaten :
**AT BE CH DE ES FR GB IT LI NL SE**

(56) Entgegenhaltungen :
**EP-A- 0 033 392**

(56) Entgegenhaltungen :
**SYNTHETIC METHODS OF ORGANIC CHEMISTRY, Band 23, 1969, Seite 280, Zusammenfassung Nr. 605, S. Karger AG, Verlag, Basel, CH**
**JOURNAL OF THE AMERICAN CHEMICAL SOCIETY, Band 77, Nr. 5, 12. März 1955, Seiten 1079-1105, American Chemical Society, Easton, PA, US; L.J. EXNER et al.: "Reactive nitrile groups. I. The reaction of alpha,omega-dinitriles with aqueous amines"**

(73) Patentinhaber : **Degussa Aktiengesellschaft**
**Weissfrauenstrasse 9**
**W-6000 Frankfurt am Main 1 (DE)**

(72) Erfinder : **Drauz, Karlheinz, Dr.**
**Zur Marienruhe 13**
**W-6463 Freigericht 1 (DE)**
Erfinder : **Krimmer, Hans-Peter, Dr.**
**Am Weissen Turm 48**
**W-6000 Frankfurt 60 (DE)**

**EP 0 304 017 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Salzen der allgemeinen Formel

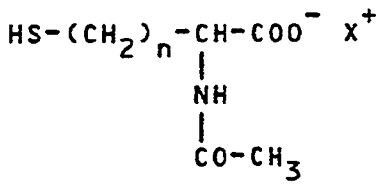

in der n die Zahl 1 oder 2 und $X^+$ ein Alkalimetallion, ein Äquivalent eines Erdalkalimetallions, ein Ammoniumion oder ein Protoniumsalz einer organischen Base bedeuten.

Salze des N-Acetylcysteins und -homocysteins sind bekannt. Sie sind von Interesse als Zwischenprodukte für die Peptidsynthese, als pharmazeutische Wirkstoffe oder als kosmetische Präparationen. Beispielsweise wird das Natriumsalz und das Ammoniumsalz von N-Acetyl-L-cystein als Mucolytikum eingesetzt.

Die Herstellung von Salzen des N-Acetylcysteins und -homocysteins stößt insofern auf Schwierigkeiten, als die selektive N-Acetylierung der Mercapto-α-aminosäuren nur unter beträchtlichen Umständen möglich ist. Durch das erfindungsgemäße Verfahren können jedoch Salze des N-Acetylcysteins und -homocysteins auf ganz einfache Weise direkt hergestellt werden.

Das erfindungsgemäße Verfahren ist dadurch gekennzeichnet, daß man Acetonitril in einem mit einer das Kation $X^+$ liefernden Base auf einen pH-Wert im Bereich von 8 bis 10 eingestellten wäßrigen oder wäßrig-organischen Medium bei einer Temperatur im Bereich von 0°C bis zur Siedetemperatur des Reaktionsgemisches mit Cystein oder Homocystein umsetzt und nach beendeter Umsetzung das Lösungsmittel unter vermindertem Druck abdampft.

Es ist zweckmäßig, das Acetonitril in einem 3- bis 5-fachen molaren Überschuß einzusetzen, da sich dadurch eine Verkürzung der erforderlichen Reaktionszeit erzielen läßt. Auch bei der eingesetzten Base empfiehlt sich ein geringer molarer überschuß von etwa 10 bis 20%.

Die Umsetzung zwischen dem Acetonitril und der Mercapto-α-aminosäure wird vorzugsweise so vorgenommen, daß man das Reaktionsgemisch unter Rückfluß zum Sieden erhitzt. Sie erfordert dann im allgemeinen eine Reaktionszeit zwischen 2 und 8 Stunden.

Es ist zweckmäßig, die Umsetzung in einer Stickstoffatmosphäre vorzunehmen, um die Gefahr einer Oxidation der Mercaptoverbindungen zu den entsprechenden Disulfiden hintanzuhalten. Besonders vorteilhaft ist es, den Stickstoff während der gesamten Umsetzung durch das Reaktionsgemisch hindurchzuleiten, um gleichzeitig den entstehenden Ammoniak auszutreiben, wodurch unter Umständen die erforderliche Reaktionszeit verkürzt wird.

Als das Kation $X^+$ liefernde Basen kommen beispielsweise in Frage : Alkali- oder Erdalkalimetallhydroxide, wie Lithium-, Natrium-, Kalium-, Magnesium- oder Calciumhydroxid ; Alkali-oder Erdalkalimetallcarbonate oder -hydrogencarbonate, wie Lithium-, Natrium-, Kalium-, Magnesium- oder Calciumcarbonat oder -hydrogencarbonat ; Ammoniak ; Alkyl-, Dialkyl- oder Trialkylamine, wie Methyl-, Ethyl-, Propyl-, Isopropyl-, n-Butyl-, Isobutyl-, sec-Butyl-, tert-Butyl-, Dimethyl-, Diethyl-, Di-n-propyl-, Diisopropyl-, Di-n-butyl-, Diisobutyl-, Di-sec-butyl-, Trimethyl-, Triethyl-, Tri-n-propyl-, Diisopropylmethyl- oder Diisopropylethylamin ; oder ungesättigte heterocyclische Basen, wie Pyridin, α-, β- und γ-Picolin, Lutidin, Chinolin, Isochinolin, Collidin, Pyrrolidin, Piperidin, Piperazin, N-Methylpiperidin oder Chinuclidin.

Sofern die anorganischen Basen oder leicht wasserlösliche organische Basen eingesetzt werden, genügt reines Wasser als Reaktionsmedium. Bei Einsatz von weniger wasserlöslichen organischen Basen empfiehlt sich die Verwendung eines wäßrig-organischen Reaktionsmediums, also einer Mischung aus Wasser und einem mit Wasser zumindest teilweise mischbaren organischen Lösungsmittel. Geeignete Lösungsmittel sind beispielsweise aliphatische Alkohole mit 1 bis 4 Kohlenstaffatomen und Ether, wie Tetrahydrofuran oder 1,4-Dioxan.

Die Durchführung des erfindungsgemäßen Verfahrens kann beispielsweise so erfolgen, daß man alle Ausgangsstoffe in Wasser oder einem geeigneten wäßrig-organischen Medium löst und das Reaktionsgemisch so lange unter Rückfluß zum Sieden erhitzt, bis sich in einer mittels Hochdruckflüssigkeitschromatographie untersuchten Probe keine nicht umgesetzte Mercapto-α-aminosäure mehr nachweisen läßt. Dann wird das Lösungsmittel unter vermindertem Druck abgedampft, bis das gewünschte Salz auszukristallisieren beginnt. Nach dem Abkühlen werden die ausgeschiedenen Kristalle abgetrennt und getrocknet.

Falls beim Abdampfen des Lösungsmittels keine Kristallisation eintritt, kann sie dadurch bewirkt werden, daß man den Rückstand abkühlt und mit einem geeigneten Lösungsmittel, beispielsweise Ethanol, digeriert.

Bei der Umsetzung von optisch aktivem Cystein oder Homocystein weist das erfindungsgemäße Verfahren den Vorteil auf, daß die Umsetzung unter Erhalt des Chiralitätszentrums verläuft.

Die Erfindung wird durch die nachfolgenden Beispiele näher erläutert :

Beispiel 1 :

12,3 g (0,3 Mol) Acetonitril wurden in 250 ml Wasser unter Durchleiten von Stickstoff eine Stunde lang zum Sieden erhitzt. Dann wurde auf 60°C abgekühlt und es wurden 12,1 g (0,1 Mol) L-Cystein und 8,7 g (0,126 Mol) 25 gewichtsprozentiger wäßriger Ammoniak zugegeben. Das Reaktionsgemisch wurde unter leichtem Durchleiten von Stickstoff 5 Stunden lang unter Rückfluß zum Sieden erhitzt. Anschließend wurden alle flüchtigen Anteile des Reaktionsgemisches unter vermindertem Druck abgedampft. Der Rückstand wurde mit 10 ml Ethanol digeriert und die gebildeten Kristalle wurden abgesaugt und getrocknet. Es ergaben sich 15,8 g (87% der Theorie) an farblosem Ammoniumsalz des N-Acetyl-L-cysteins mit einem Schmelzpunkt von 148-149°C (Zersetzung).

$[\alpha]_D^{25}$ = 20,3° (c = 5 ; $H_2O$)

$^1$H-NMR (DMSO-$d^6$) : δ = 1,87 (s ; 3H, Acetyl-$CH_3$), 2,76 (mc ; 2H, β-$CH_2$), 4,02 (m ; 1H, α-H), 6,34 (breit; 5H, $NH_4^+$ und SH), 7,50 (d ; 1H, NH).

Beispiel 2 :

20,5 g (0,5 Mol) Acetonitril, 12,1 g (0,1 Mol) L-Cystein und 6,9 g (0,05 Mol) Kaliumcarbonat wurden unter Stickstoff in 250 ml Wasser 4 Stunden lang unter Rückfluß zum Sieden erhitzt. Anschließend wurde Kohlendioxid durch das Reaktionsgemisch geleitet und es wurde weitere 30 Minuten lang erhitzt. Das Reaktionsgemisch wurde unter vermindertem Druck solange eingeengt, bis die Kristallisation begann. Nach dem Abkühlen wurden die ausgeschiedenen Kristalle abgesaugt und getrocknet. Es ergaben sich 18,2 g (91% der Theorie) an farblosem Kaliumsalz des N-Acetyl-L-cysteins.

Beispiel 3 :

20,5 g (0,5 Mol) Acetonitril, 12,1 g (0,1 Mol) L-Cystein und 4,4 g (0,11 Mol) Natriumhydroxid wurden unter Stickstoff in 250 ml Wasser unter Rückfluß zum Sieden erhitzt. Der Umsatz wurde dabei mittels Hochdruckflüssigkeitschromatographie verfolgt. Nach beendeter Umsetzung wurde so weiterverfahren wie im Beispiel 2. Es ergaben sich 14,6 g (80% der Theorie) an farblosem Natriumsalz des N-Acetyl-L-cysteins.

## Patentansprüche

1. Verfahren zur Herstellung von Salzen der allgemeinen Formel

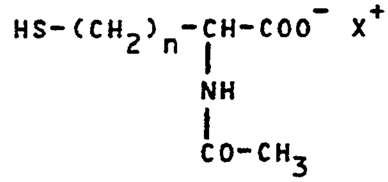

in der n die Zahl 1 oder 2 und X⁺ ein Alkalimetallion, ein Äquivalent eines Erdalkalimetallions, ein Ammoniumion oder ein Protoniumsalz einer organischen Base bedeuten, dadurch gekennzeichnet, daß man Acetonitril in einem mit einer das Kation X⁺ liefernden Base auf einen pH-Wert im Bereich von 8 bis 10 eingestellten wäßrigen oder wäßrig-organischen Medium bei einer Temperatur im Bereich von 0°C bis zur Siedetemperatur des Reaktionsgemisches mit Cystein oder Homocystein umsetzt und nach beendeter Umsetzung das Lösungsmittel unter vermindertem Druck abdampft.

2. Verfahren nach Anspruch 1, dadurch gekennzeichnet, daß man die Umsetzung in einer Stickstoffatmosphäre vornimmt.

3. Verfahren nach Anspruch 2, dadurch gekennzeichnet, daß man den Stickstoff während der Umsetzung durch das Reaktionsgemisch hindurchleitet.

## Claims

1. A process for the production of salts corresponding to the following general formula

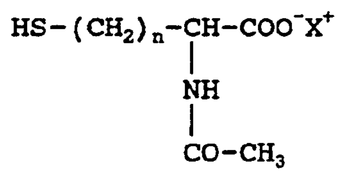

in which n is the number 1 or 2 and X⁺ is an alkali metal ion, an equivalent of an alkaline earth metal ion, an ammonium ion or a protonium salt of an organic base, characterized in that acetonitrile is reacted with cysteine or homocysteine in an aqueous or aqueous-organic medium adjusted to a pH value of 8 to 10 with a base yielding the cation X⁺ at a temperature in the range from 0°C to the boiling temperature of the reaction mixture and, on completion of the reaction, the solvent is evaporated off under reduced pressure.

2. A process as claimed in claim 1, characterized in that the reaction is carried out in a nitrogen atmosphere.

3. A process as claimed in claim 2, characterized in that nitrogen is passed through the reaction mixture during the reaction.

## Revendications

1. Procédé d'obtention de sels de formule générale

$$HS-(CH_2)_n-CH-COO^-\ X^+$$

$$|$$

$$NH$$

$$|$$

$$CO-CH_3$$

dans laquelle n signifie le nombre 1 ou 2 et $X^+$ signifie un ion de métal alcalin, un équivalent d'ion de métal alcalino-terreux, un ion ammonium ou un sel de protonium d'une base organique, caractérisé en ce que l'on fait réagir l'acétronitrile dans un milieu aqueux ou hydro-organique ajusté à un pH dans la zone de 8 à 10 avec une base qui fournit le cation $X^+$ à une température dans la zone allant de 0° à la température d'ébullition du mélange réactionnel, avec la Cystéine ou l'homocystéine et que l'on chasse le solvant par distillation sous pression réduite après achèvement de la réaction.

2. Procédé selon la revendication 1, caractérisé en ce que l'on effectue la réaction en atmosphère d'azote.

3. Procédé selon la revendication 2, caractérisé en ce que l'on fait passer l'azote pendant la réaction à travers le mélange réactionnel.